# FASCICULE DE BREVET EUROPEEN

(11) **EP 0 686 637 B1**
(45) Date de publication et mention de la délivrance du brevet: **26.04.2000**
(21) Numéro de dépôt: 95401311.6
(22) Date de dépôt: 07.06.1995
(51) Int. Cl.: C07D 498/04, C07D 498/14, A61K 31/535, C07D 491/048, C07D 491/153

(54) **Dérivés tétracycliques de la 1,4-oxazine, leur procédé de préparation et les compositions pharmaceutiques les contenant**
Tetracyclische 1,4-Oxazin-Derivate, Verfahren zu ihrer Herstellung und diese enthaltende pharmazeutische Zubereitungen
Tetracyclic 1,4-oxazine derivatives, process for their preparation and pharmaceutical compositions containing them

(30) Priorité: 08.06.1994 FR 9406985
(43) Date de publication de la demande: 13.12.1995
(73) Titulaire: ADIR ET COMPAGNIE, 92415 Courbevoie Cédex (FR); INSTITUT NATIONAL DE LA SANTE ET DE LA RECHERCHE MEDICALE (INSERM), 75654 Paris Cédex 13 (FR)
(72) Inventeur: Peglion, Jean-Louis, F-78110 le Vesinet (FR); Vian, Joel, F-92370 Chaville (FR); Goument, Bertrand, F-78220 Viroflay (FR); Millan, Mark, F-75017 Paris (FR); Audinot, Valérie, F-78300 Croissy (FR); Schwartz, Jean-Charles, F-75014 Paris (FR); Sokoloff, Pierre, F-95130 Le Plessis Bouchard (FR)
(74) Mandataire: Reverbori, Marcelle

(56) Documents cités:
- EP-A- 0 080 115
- EP-A- 0 161 218
- EP-A- 0 246 633
- JOURNAL OF MEDICINAL CHEMISTRY., vol.27, no.12, Décembre 1984, WASHINGTON US pages 1607 - 1613 J. H. JONES ET AL 'Synthesis of 4-substituted 2H-naphth(1,2-b)-1,4-oxazines, a new class of dopamine agonists'

## Description

La présente invention a pour objet de nouveaux dérivés tétracycliques de la pipérazine et de l'oxazine, leur procédé de préparation et les compositions pharmaceutiques les contenant.

Elle concerne plus particulièrement les composés de formule générale **(I)**. 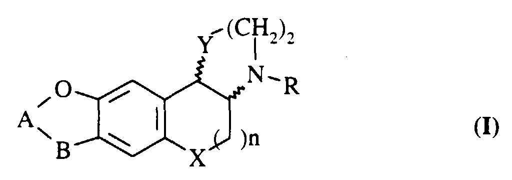 dans laquelle :
- X et Y, identiques ou différents, représentent chacun un atome d'oxygène ou CH₂ ;
- A-B représente -(CH₂)₂- ou -HC=CH-, et de plus :
   . lorsque Y représente un atome d'oxygène, A-B peut aussi représenter -(CH₂)₃- et
   . lorsque Y représente CH₂, A-B peut aussi représente 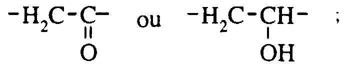
- R représente un atome d'hydrogène ou un radical (C₁-C₁₀) alkyle, (C₃-C₁₀) alkényle ou (C₃-C₁₀) alkinyle, chacun pouvant être en chaîne droite ou ramifiée et chacun pouvant être substitué par un radical cycloalkyle ayant de 3 à 8 atomes de carbone, ou par un radical aryle choisi parmi les radicaux phényle, thiényle et pyridyle, chacun d'eux éventuellement substitué par un ou plusieurs substituants choisis parmi les atomes d'halogène le radical hydroxy et les radicaux alkyle et alkoxy ayant chacun de 1 à 6 atomes de carbone en chaîne droite ou ramifiée ; et
- n représente :
   . zéro ou 1 lorsque X représente -CH₂ et
   . uniquement 1 lorsque X représente un atome d'oxygène.

Pour les produits de l'invention, il existe la possibilité de deux formes isomères géométriques cis et trans. Ces deux formes sont incluses dans la présente invention. De même la présence de carbones asymétriques implique que les molécules de l'invention existent sous la forme de mélange racémique ou racémate et d'isomères optiques ou énantiomères, également inclus dans la présente invention. De plus les composés de l'invention peuvent former avec des acides pharmaceutiquement acceptables des sels d'acides organiques ou inorganiques, qui font aussi partie de la présente invention.

L' état antérieur de la technique le plus proche de la présente invention concerne :
- des composés du benzopyrane de structure : 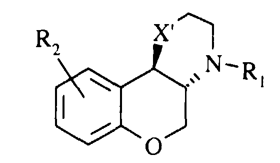 dans laquelle : :
   . X' représente un atome d'oxygène (EP 0246633) ou
   . X' représente -CH₂- (EP 0161218),
- ainsi que des composés de structure : 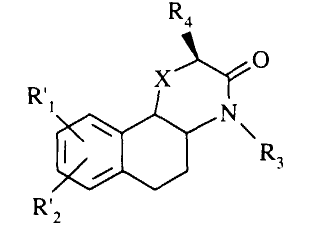 décrits dans la demande WO 93 24471
utilisés dans le traitement des maladies du système nerveux central telles que la schizophrénie ou la maladie de Parkinson.

Ces substances exercent leur action au niveau des récepteurs dopaminergiques D₂ et en ce sens induisent des effets secondaires gênants tels que : augmentation de la sécrétion de prolactine, rigidité musculaire ou ralentissement moteur et, après un traitement prolongé, survenue de mouvements anormaux involontaires (dyskinésies tardives).

Récemment P. Sokoloff et al. (Nature, 1990, 347, 147) ont mis en évidence l'existence d'un nouveau récepteur dopaminergique appelé D₃. Sa forte concentration au niveau du système limbique et sa faible densité au niveau des cellules lactotrophes et dans le système nigrostrié en fait une cible de choix pour l'obtention d'antipsychotiques dépourvus d'effet sur la sécrétion de prolactine et moins susceptibles de provoquer des syndromes de type extrapyramidaux.

Les études réalisées in vitro (binding sur récepteurs clonés D₂ et D₃ humains et de rats) avec les composés de l'invention montrent que ceux-ci se comportent comme des ligands très affins au niveau des récepteurs dopaminergiques D₃, tout en ne montrant que peu d'affinité pour les récepteurs dopaminergiques D₂. La grande sélectivité des produits de l'invention pour les récepteurs D₃ vis à vis des récepteurs D₂ permet donc d'envisager, pour les produits de l'invention, une nette diminution des effets secondaires rapportés avec les substances spécifiquement D₂.

Cette sélectivité confère aux produits de l'invention un intérêt tout particulier pour leur utilisation comme médicaments agissant au niveau du système dopaminergique, car ils ne devraient pas provoquer les effets indésirables des ligands D₂.

Les composés de la présente invention se différencient donc des composés de l'état antérieur de la technique non seulement par leur structure chimique mais aussi au niveau de leur activité pharmacologique et thérapeutique.

Les études réalisées in vivo ont permis :
1°) de mettre en évidence chez l'animal l'activité antagoniste des produits de l'invention au niveau des récepteurs D₃ (réversion de l'hypothermie induite par l'agoniste prototypique D₃ : 7-OH-DPAT) selon la méthode de M.J. Millan (Eur. J. Pharmacol., 1994, 260, R₃-R₅).
2°) de démontrer pour la première fois l'utilité de tels produits dans le traitement de la dépression, en utilisant le test bien connu de la nage forcée publié en 1978 par Porsolt. (Eur. J. Pharmacol, 47, 379-391).

De plus il est généralement admis d'après la littérature que les produits agissant de préférence au niveau des récepteurs dopaminergiques D₃ peuvent être utilisés dans le traitement de l'abus de drogue (B. Caine, Science, 1993, 260, 1814), comme anti-parkinsonnien (Carlsson, J. Neur. Transm. , 1993, 94, 11-19), antipsychotique et dans les troubles de la mémoire (P. Sokoloff, op. cit.).

Les produits de l'invention possèdent donc des propriétés pharmacologiques et thérapeutiques très intéressantes et se présentent comme des médicaments des troubles du système nerveux central, pouvant être utilisés comme antidépresseurs, anti-psychotiques, anti-parkinsoniens et anti-mnésiques. Ils conviennent également pour le traitement des troubles liés à l'abus de drogue.

L'invention s'étend également au procédé de préparation des composés de l'invention appartenant à la famille de la 1,4-oxazine, c'est à dire des composés de formule **I** dans laquelle Y représente un atome d'oxygène,
caractérisé en ce que l'on réduit un composé de formule **II** : 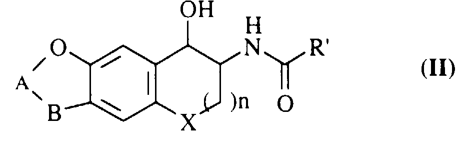 dans laquelle :
- A-B, X et n ont les significations précédemment définies, et
- R' représente un radical phényle, thiényle ou pyridyle, chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée, un radical cycloalkyl ayant de 3 à 8 atomes de carbone, un radical (C₁-C₉)alkyle, (C₂-C₉)alkényle ou (C₂-C₉)alkynyle, chacun pouvant être en chaîne droite ou ramifiée et chacun pouvant être substitué par un radical cycloalkyle ayant de 3 à 8 atomes de carbone, ou par un radical aryle choisi parmi les radicaux phényle, thiényle et pyridyle, chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée,
pour obtenir un composé de formule **III** : 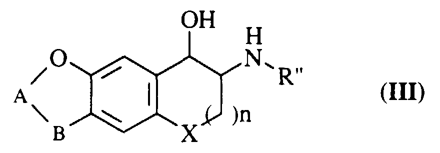 dans laquelle :
- A-B, X et n ont les significations précédemment définies, et
- R" prend la signification de R à l'exception des valeurs hydrogène et méthyle ;
lequel dérivé **III** est traité par un halogènure d'acide de formule **IV** : 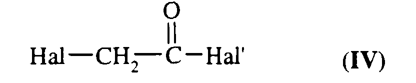 dans laquelle Hal et Hal' identiques ou différents, représentent chacun un atome de chlore ou de brome,
pour obtenir un composé de formule **V**: 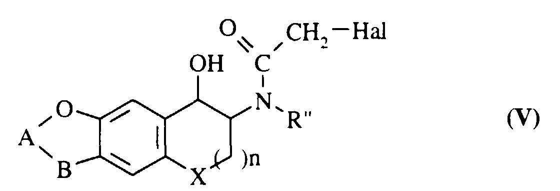 dans laquelle A-B, X, n, R" et Hal ont les significations précédemment définies ; que l'on traite avec un hydrure de métal alcalin, tel que par exemple l'hydrure de sodium, pour obtenir un composé de formule **VI** : 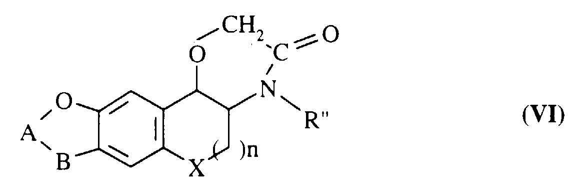 dans laquelle A-B, X, n et R" ont les significations précédemment définies ; que l'on traite par un hydrure double de lithium et d'aluminium pour obtenir un composé de formule **Ia** : 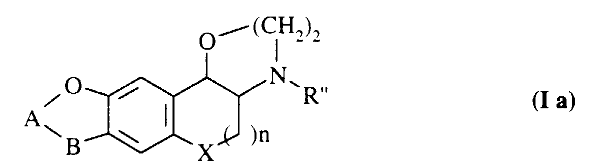 dans laquelle A-B, X, n et R" ont les significations précédemment définies,
et dans le cas où R" prend la valeur benzyle,
on débenzyle le composé correspondant de formule **Ia'** : 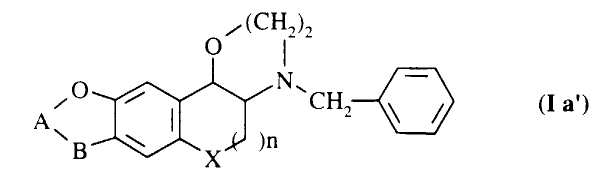 dans laquelle A-B, X et n ont les significations précédemment définies ;
pour obtenir un composé de formule **Ib** : 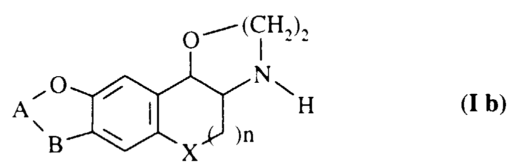 dans laquelle A-B, X et n ont les significations précédemment définies ;
qui est à son tour traité par un agent de méthylation pour obtenir un composé de formule **Ic** : 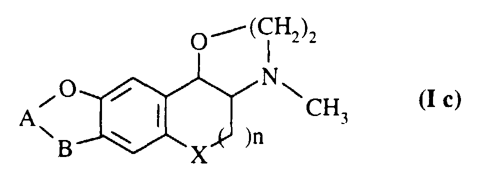 dans laquelle A-B, X et n ont les significations précédemment définies.

Alternativement, les composés de formule **I** dans laquelle A-B représente -HC=CH-, c'est à dire les composés répondant à la formule : 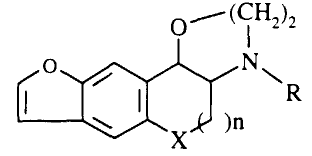 dans laquelle X, n et R ont les significations précédemment définies,
peuvent également être préparés par oxydation des composés correspondants de formule **I** dans laquelle A-B représente (CH₂)₂.

(Cette oxydation s'effectuant avantageusement, par exemple, au moyen de 2,3-dichloro 5,6-dicyano 1,4-benzoquinone dans l'acide acétique).

L'ensemble des composés de formule **Ia**, **Ib** et **Ic** forment l'ensemble des composés de formule **I** dans laquelle Y représente un atome d'oxygène.

La réduction des composés de formule **II** s'effectue avantageusement au moyen d'un hydrure de lithium et d'aluminium, dans un solvant adéquat tel que, par exemple, le tétrahydrofurane.

La débenzylation des composés de formule **Ia'** s'effectue de manière particulièrement satisfaisante par action de l'hydrogène, avec ou sans pression, en présence des catalyseurs classiques d'hydrogénation, tel que, par exemple, Pd/C.

La méthylation des composés de formule **Ib** est réalisée de façon particulièrement adéquate au moyen d'un halogénure de méthyle, du sulfate de méthyle, du phosphate de méthyle ou du formol dans l'acide formique.

En ce qui concerne les composés de la 1,4-oxazine, les alcools de départ de formule **II** peuvent exister sous deux formes isomères géométriques cis et trans de formules **II cis** et **II trans.** 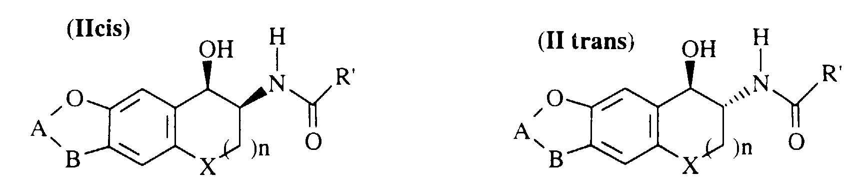 dans lesquelles A-B, X, R' et n ont les significations précédemment définies.

L'ensemble des composés de formules **II cis** et **II trans** forme l'ensemble des composés de formule **II**.

Les composés **II trans** sont obtenus à partir des amino-cétones de formule **VII** : 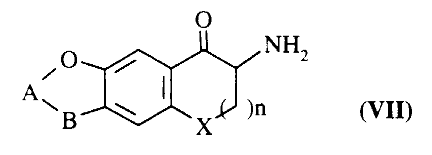 dans laquelle A-B, X et n prennent les valeurs précédemment définies, qui sont soumis à une réaction de Schotten-Baumann en présence d'un halogénure d'acide de formule **VIII** : 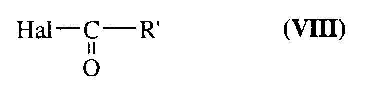 dans laquelle R' et Hal ont les significations précédemment définies, pour obtenir des composés de formule **IX** : 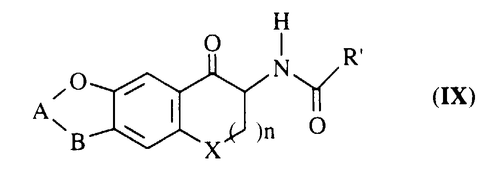 dans laquelle A-B, X, n et R' prennent les valeurs précédemment définies,
qui sont réduits par un borohydrure de métal alcalin pour conduire aux composés **II trans**.

Les amino-cétones de formule (**VII**) sont des substances connues, ou peuvent être obtenues par des méthodes connues à partir de substances connues.

Les composés **II cis** sont obtenus à partir des azido-cétones de formule **X** : 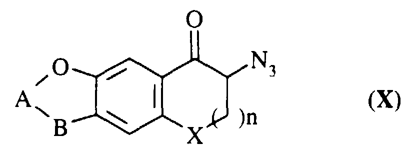 dans laquelle A-B, X et n ont les significations précédemment définies,
qui, réduits par l'hydrure de lithium et d'aluminium, conduisent aux amino alcools de géométrie cis de formule **XI** : 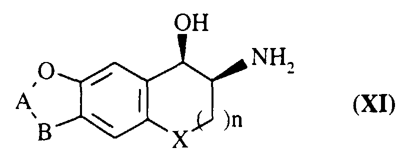 dans laquelle A-B, X et n ont les significations précédemment définies,
qui sont soumis à l'action d'un halogénure d'acide de formule **VIII**, pour conduire aux composés **II cis**.

Les azido-cétones de formule **X** sont des substances connues ou sont formées par des méthodes connues à partir de substances connues, comme indiqué ci-après dans le chapitre préparation des matières premières.

De même les composés de formule **III**, **V**, **VI**, **Ia**, **Ia'**, **Ib** et **Ic** existent sous les formes géométriques cis et trans.

L'invention s'étend également au procédé de préparation des composés de l'invention appartenant à la famille de la pipéridine, c'est à dire des composés de formule I dans laquelle Y représente CH₂,
caractérisé en ce aue l'on réduit un comDosé de formule **XII** : 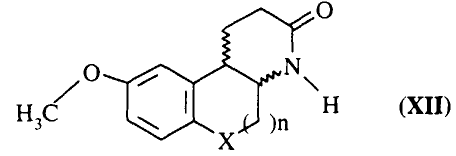 dans laquelle X et n ont les significations précédemment définies ;
pour obtenir un composé de formule **XIII** : 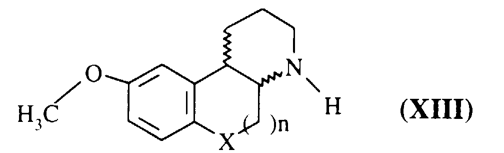 dans laquelle X et n ont les significations précédemment définies ;
lequel composé est traité par un halogénure d'acide de formule **VIII** : 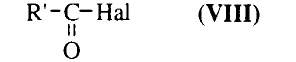 dans laquelle R' et Hal ont les significations précédemment définies.
pour obtenir un composé de formule **XIV** : 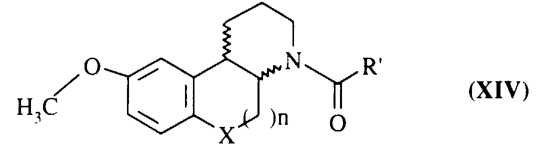 dans laquelle X, n et R' ont les significations précédemment définies,
que l'on soumet à l'action du tribromure de bore dans le chloroforme pour obtenir un composé de formule **XV** : 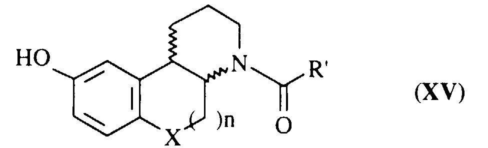 dans laquelle X, n et R' ont les significations précédemment définies
que l'on alcoyle par l'intermédiaire d'une réaction de Friedel et Crafts pour obtenir un composé de formule **XVI** : 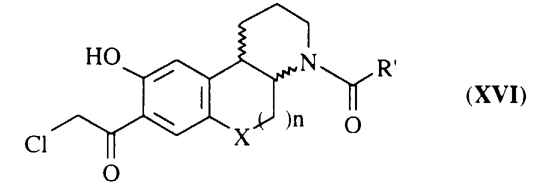 dans laquelle X, n et R' ont les significations précédemment définies,
que l'on cyclise en milieu basique pour obtenir un composé de formule **XVII** : 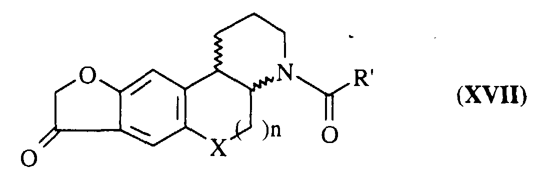 dans laquelle X, n et R' ont les significations précédemment définies,
qui est réduit par du borohydrure de sodium, pour obtenir un composé de formule **XVIII** : 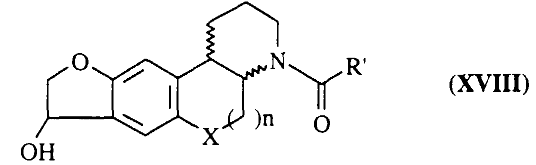 dans laquelle X, n et R' ont les significations précédemment définies,
qui est deshydraté en milieu acide pour obtenir un composé de formule **XIX** : 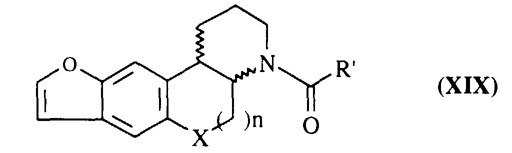 dans laquelle X, n et R' ont les significations précédemment définies,
qui est réduit par une solution toluénique d'hydrure de sodium bis(2-méthoxyéthoxy)aluminium pour obtenir un composé de formule **Id** : 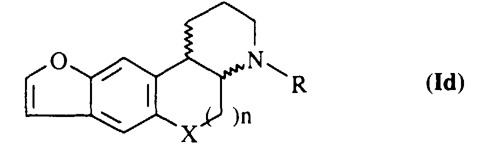 dans laquelle X, n et R ont les significations précédemment définies,
que l'on peut éventuellement réduire par l'hydrogène en présence d'un catalyseur au Pt ou au Pd sur charbon, avec ou sans pression pour obtenir un composé de formule **Ie** : 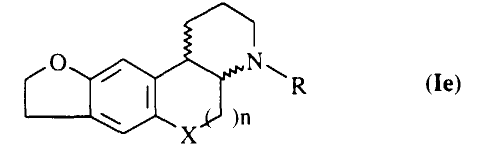 dans laquelle R, X et n ont les significations précédemment définies.

Les composés de formule **Ie** peuvent aussi éventuellement être obtenus par réduction au borane-diméthylsulfure des composés de formule **XVII**.

D'autre part, la réduction à l'hydrure d'aluminium des composés de formule **XVII** conduit aux composés de formule **If** : 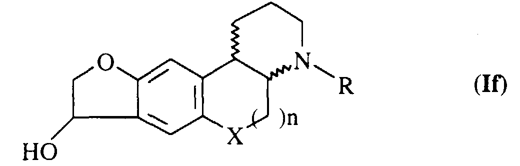 dans laquelle X, n et R ont les significations précédemment définies,
lesquelles peuvent être oxydés par l'intermédiaire d'un agent oxydant tel que l'oxyde de manganèse ou le réactif de Jones pour conduire aux composés de formule **Ig** : 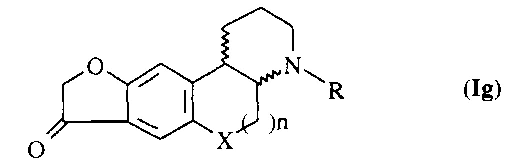 dans laquelle X, n et R ont les significations précédemment définies.

En ce qui concerne la préparation des composés appartenant à la famille de la pipéridine, les composés de formule **XII** sont réduits soit par l'hydrure de lithium et d'aluminium dans le tétrahydrofurane, soit par le borane diméthylsulfure dans le tétrahydrofurane.

L'alcoylation des composés de formule **XV** est avantageusement réalisée avec le chloroacétonitrile en présence de trichlorure de bore et de trichlorure d'aluminium dans le chlorure de méthylène.

La cyclisation des composés de formule **XVI** est réalisée par l'intermédiaire d'une amine tertiaire telle que la triéthylamine dans le chloroforme.

La déshydratation des composés de formule **XVIII** est plus avantageusement réalisée dans un acide minéral, tel que l'acide chlorhydrique.

En ce qui concerne la préparation des composés appartenant à la famille de la pipéridine, les pipéridin-ones de départ de formule **XII** peuvent exister sous deux formes isomères géométriques cis et trans de formule **XIIcis** et **XIItrans** : 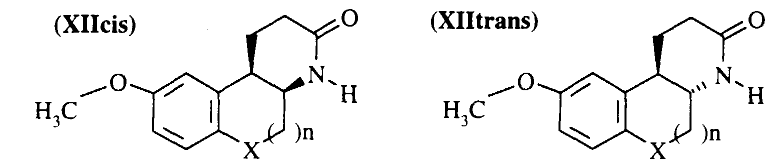 dans lesquelles X et n ont les significations précédemment définies.

L'ensemble des composés de formule **XIIcis** et **XIItrans** forme l'ensemble des composés de formule **XII**.

Les composés de formule **XIIcis** et **XIItrans** sont obtenus par réduction des composés de formule **XX** : 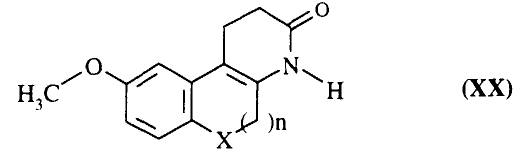 dans laquelle X et n ont les significations précédemment définies,
les composés **XX** étant quant à eux obtenus à partir des composés de formule **XXI** : 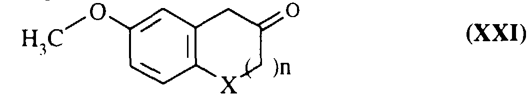 dans laquelle X et n ont les valeurs précédemment définies, par action de la pyrolidine en présence d'APTS dans le benzène à reflux suivie par un traitement par un excès d'acrylamide à 80 °C puis à 140 °C.

Les composés de formule **XII trans** sont obtenus par réduction des composés de formule **XX** par le triéthylsilane dans le chlorure de méthylène en présence d'acide trifluoroacétique et les dérivés **XIIcis** , sont obtenus par réduction catalytique en présence de Pt/C des composés de formule **XX**, après séparation chromatographique du mélange réactionnel composé de 62 % du composé **XIIcis** et 36 % du composé **XIItrans**.

De même, les composés de formule **XIII**, **XIV**, **XV**, **XVI**, **XVII**, **XVIII**, **XIX**, **Id**, **Ie**, **If** et **Ig** existent sous les formes géométriques cis et trans.

Les exemples suivants illustrent l'invention mais ne la limitent en aucune façon.

Les points de fusion sont déterminés soit à la platine chauffante de Köfler (K), soit à la platine chauffante sous microscope (M. K). Les spectres de résonance magnétique nucléaire du proton (RMN) ont été réalisés à 200 MHz (sauf indications contraires) en utilisant le tétraméthylsilane (TMS) comme référence interne. Les déplacements chimiques sont exprimés en partie par million. (ppm).

### Exemple 1:

### trans-3,4,4a,5,6,8,9,11b-octahydro furo [2,3-b] 1,4-oxazino [3,2-h] 4-propyl-2H- naphtalène.

### Stade A: N-(8-oxo 2,3,6,7-tétrahydro -5H- naphto [2,3-b] furan -7 yl) propionamide.

A 17 g de chlorhydrate de dl -7-amino 2,3,6,7-tétrahydro-*5H*-naphto [2,3-b] furan 8-one en suspension dans 170 ml de chlorure de méthylène sont ajoutés successivement 23 ml de triéthylamine et 5,7 ml de chlorure de propionyle. Après 2 h d'agitation à température ambiante le milieu réactionnel est lavé à l'eau, décanté et concentré à siccité. On obtient 20 g du composé désiré.
P.F. : 162-164°C (K) Rendement : 100%

### Stade B : trans-N-(8-hydroxy-2,3,5,6,7,8-hexahydro naphto [2,3-b] furan-7-yl) propionamide.

3 g de borohydrure de sodium sont ajoutés par portion à une solution de 20 g du composé obtenu précédemment dans 400 ml d'éthanol. Lorsque la réaction est terminée (c.c.m.) le mélange réactionnel est concentré sous vide, repris à l'eau et extrait à l'acétate d'éthyle. La phase organique séchée sur sulfate de magnésium est concentrée sous vide pour donner 13 g du composé désiré.
P.F. : 182-184°C (K) Rendement : 65%
R.M.N.¹H (DMSO d6): 6,9 ppm, s, 1H ; 6,8 ppm, s, 1H ; 5,2 ppm 1H échangeable ; 4,45 ppm, t, 2H ; 4,3 ppm, t, 1H (J ≃ 7,9 Hz); 3,8 ppm, m, 1H (J ≃ 7,9 Hz) ; 3,1 ppm, t, 2H ; 2,7 ppm, t, 2H ; 2,15 ppm, q, 2H ;1,95 à 1,6 ppm, 2m, 2H ; 1 ppm, t, 3H.

### Stade C : trans-N-(8-hydroxy-2,3,5,6,7,8-hexahydro naphto [2,3-b] furan-7-yl) propylamine

13 g du composé obtenu précédemment en solution dans 130 ml de tétrahydrofurane sont ajoutés goutte à goutte à une suspension de 4,7 g d'hydrure de lithium et d'aluminium dans 60 ml de tétrahydrofurane. Après 18 h à température ambiante le milieu réactionnel est hydrolysé par 3,12 ml d'eau, suivi de 2,5 ml de soude à 20 % suivi de 11,5 ml d'eau. Après filtration des sels minéraux et concentration du filtrat sous vide, 11,7 g du composé désiré sont obtenus.
P.F. : 144-146°C (K) Rendement : 94%

### Stade D : trans-N-(8-hydroxy-2,3,5,6,7,8-hexahydro naphto[2,3-b]furan-7 yl)N-propyl 2-chloroacétamide.

A 11,5 g du composé obtenu précédemment, dissous dans 600 ml d'acétate d'éthyle, sont ajoutés 300 ml d'une solution saturée de carbonate de sodium suivi de 3,8 ml de chlorure de chloroacétyle. Quand la réaction est complète (c.c.m.), le milieu réactionnel est décanté, séché sur sulfate de magnésium, filtré et concentré sous vide.

19 g de ce composé sont obtenus.
P.F. : 65-70°C (K) Rendement:100%

### Stade E: trans-4a,5,6,8,9,11b-hexahydro furo[2,3-b] 1,4-oxazino[3,2-h] 3-oxo 4-propyl-2H-naphtalène.

Une solution de 19 g du composé obtenu précédemment dans un mélange de 76 ml d'acétonitrile et de 380 ml de tétrahydrofurane est ajoutée goutte à goutte à une suspension de 6,7 g d'hydrure de sodium (à 50 % dans l'huile) dans 50 ml de tétrahydrofurane. Lorsque la réaction est terminée, l'excès d'hydrure est décomposé avec de l'éthanol, le milieu réactionnel est concentré à siccité, repris à l'eau, extrait à l'acétate d'éthyle. La phase organique séchée sur sulfate de magnésium est concentrée sous vide pour donner 8,5 g du composé désiré.
P.F. : > 260°C (K) Rendement : 53%

### Stade F : Composé titre de l'exemple.

8,5 g du composé obtenu précédemment sont traités dans les mêmes conditions que celles utilisées pour le stade C. On obtient 2,5 g du composé désiré après recristallisation de l'acétate d'éthyle.
P.F. : 92-94°C (K) Rendement : 34%
R.M.N. ¹H (DMSO d6)

6,9 ppm, s, 1H ; 6,7 ppm, s, 1H ; 4,5 ppm, t, 2H ; 4,1 ppm, d, 1H (J ≃ 8,3 Hz) ; 3,95 ppm, dd 1H ; 3,75 ppm, td, 1H ; 3,15 ppm, t, 2H ; 2,8 ppm, m, 4H ; 2,3 à 2,15 ppm, m, 3H ; 2,05 ppm, m, 1H (J ≃ 8,3 Hz) ; 1,5 ppm, m, 3H ; 0,85 ppm, t, 3H.

### Exemple 2:

### trans-3,4,4a,5,6,8,9,11b-octahydro furo[2,3-b] 1,4-oxazino[3,2-h] 4-(2-phényléthyl)-2H-naphtalène.

### Stade A : N-(8-oxo-2,3,6,7-tétrahydro-5H-naphto[2,3b] furan-7-yl) phénylacétamide.

Ce composé est obtenu en utilisant la méthode décrite au stade A de l'exemple 1 mais en remplaçant le chlorure de propionyle par le chlorure de l'acide phénylacétique.
P.F. : 171-173°C (K) Rendement : 100%

### Stade B : trans-N-(8-hydroxy-2,3,5,6,7,8-hexahydro-naphto[2,3-b] furan-7-yl) benzylamide.

Ce composé est obtenu en appliquant au composé ci-dessus la méthode décrite au stade B de l'exemple 1.
P.F. : 148-150°C (K) Rendement : 75%
R.M.N. ¹H (CDCl₃).

7,3 ppm, m, 5H ; 6,95 à 6,8 ppm, 2s, 2H ; 4,5 ppm, t+m, 3H, (J≃ 7,9 Hz) ; 4,0 ppm, m, 1H ; (J ≃ 7,9 Hz) ; 3,6 ppm, s, 2H ; 3,15 ppm, t, 2H ; 2,9 ppm, m, 2H ; 2,15 à 1,7 ppm, m, 2H.

### Stade C: trans-N-(8-hydroxy-2,3,5,6,7,8-hexahydro-naphto[2,3-b]furan-7-yl)2-phényl éthylamine.

En suivant le mode opératoire du stade C de l'exemple 1, on obtient le composé désiré à partir du composé ci-dessus.
P.F. : 144-148°C (K) Rendement : 67%

### Stade D: trans-N-(8-hydroxy-2,3,5,6,7,8-hexahydro-naphto[2,3-b]furan-7-yl) N-(2-phényléthyl-amine) 2-chloroacétamide.

Composé obtenu sous forme de meringue selon le stade D de l'exemple 1 à partir du composé ci-dessus.
Rendement:100%

### Stade E : trans-4a,5,6,8,9,11b-hexahydro-furo[2,3-b] 1,4-oxazino[3,2-h]3-oxo 4-(2-phényl éthyl) -2H-naphtalène.

Composé obtenu selon le stade E de l'exemple 1 à partir du composé ci-dessus.
P.F. : 214-216°C (K) Rendement : 34%

### Stade F: Composé titre de l'exemple.

En appliquant au composé ci-dessus la méthode décrite au stade F de l'exemple 1 on obtient le composé titre après recristallisation de l'éther isopropylique.
P.F. : 98-100°C (K) Rendement:10%
R.M.N. ¹H (CDCl₃).

7,4 à 7,1 ppm, m, 5H ; 6,95 à 6,85 ppm, 2s, 2H ; 4,5 ppm, t, 2H ; 4,25 ppm, d, 1H, (J≃ 8,5 Hz) ; 4,15 à 3,8 ppm, 2dd, 2H ; 3,2 à 2,5 ppm, m, 10H (J≃ 8,5 Hz) ; 2,3 ppm, m, 2H ; 1,7 à 1,5 ppm, m, 1H.

### Exemple 3:

### trans-3,4,4a,5,6,8,9,11b-octahydro-furo[2,3-b]1,4-oxazino[3,2-h]4-(cyclopropyl méthyl)-2H-naphtalène.

Ce composé est obtenu selon la méthode de l'exemple 1 mais en remplaçant au stade A le chlorure de propionyle par le chlorure de l'acide cyclopropane carboxylique.
P.F. : 98-100°C (K) Rendement : 4% (7 stades)
R.M.N. ¹H (CDCl₃).

6,95 et 6,80 ppm, 2s, 2H ; 4,5 ppm, t, 2H ; 4,25 ppm, d, 1H, (J≃ 8,5 Hz) ; 4,0 ppm, 2dd, 2H ; 3,1 ppm, m, 3H (J≃ 8,5 Hz) ; 3,0 à 2,7 ppm, m, 3H ; 2,55 ppm, t, 1H ; 2,4 à 2,1 ppm, m, 3H ; 1,55 ppm, m, 1H ; 0,9 ppm, m, 1H ; 0,55 ppm, m, 2H ; 0,15 ppm, m, 2H.

### Exemple 4:

### cis 3,4,4a,8,9,11b-hexahydro furo[2,3-g]1,4-oxazino[5,6-c]4-propyl 5H-benzopyranne

### Stade A : cis 2,3,7,8-tétrahydro 3-amino 4-hydroxy furo[2,3-g] benzopyranne

A 8,1g d'aluminohydrure de lithium en suspension dans 200 ml de THF à température ambiante, on ajoute en 1 h le 2,3,7,8-tétrahydro 3-azido 4-oxo furo[2,3-g] benzopyranne (voir préparation 2) en solution dans 300 ml de THF. Après 18 h à température ambiante, le milieu réactionnel est hydrolysé par 5,6 ml d'eau, puis 4,5 ml de soude à 20%, puis 20,4 ml d'eau. Après filtration des sels minéraux, puis concentration du filtrat à l'évaporateur, le résidu obtenu est purifié par la technique du passage acide-base pour donner 15g du composé désiré.
Rendement : 51 %

### Stade B : cis 2,3,7,8-tétrahydro 3-propionylamino 4-hydroxy furo[2,3-g] benzopyranne

2,5g du composé précédent sont traités comme décrit à l'exemple 1 stade D (en utilisant le chlorure de propionyle à la place du chlorure de 2-chloroacétyle) pour donner 1,6g du composé désiré.
P.F. : 192°C (K) Rendement : 51%
RMN 400 MHz (DMSO-d6) :

7,5 ppm, d, H1 ; 6,7 ppm, s, 1H ; 6,6 ppm, s, 1H ; 5,6 ppm, d, 1H; 4,5 ppm, m,3H ; 4,1 ppm m,1H ; 3,9 ppm d, 2H ; 3,1ppm, t, 2H ; 2,2 ppm q, 2H ;1,0 ppm, t,3H.

Mise en évidence de l'isomérie cis par l'intermédiaire d'un effet Overhauser nucléaire entre le proton à 4,5 ppm (proton porté par le carbone portant le OH) et le proton à 4,1 ppm (proton porté par le carbone portant le NH).

### Stade C: cis 2,3,7,8-tétrahydro 3-propylamino 4-hydroxy furo[2,3-g] benzopyranne

1,5g du composé précédent sont traités par l'aluminohydrure de lithium comme décrit à l'exemple 1 stade C pour donner , après flash-chromatographie, 1g du composé désiré sous forme d'une huile.
Rendement : 70%

### Stade D: cis 2,3,7,8-tétrahydro 3-(N-propyl 2-chloroacétamido) 4-hydroxy furo[2,3-g] benzopyranne

lg du composé précédent est traité par le chlorure de 2-chloroacétyle comme décrit à l'exemple 1 stade D pour donner 1,05g du composé désiré sous forme d'une meringue.
Rendement : 81 %

### Stade E : cis 4a,8,9,11b-tétrahydro furo[2,3-g] 1,4-oxazino[5,6-c] 3-oxo 4-propyl 2H,5H-benzopyranne

1g du composé précédent est traité par l'hydrure de sodium comme décrit à l'exemple 1 stade E pour donner 0,86g du composé désiré sous forme d'une meringue.
Rendement : 97%

### Stade F : cis 3,4,4a,8,9,11b-hexahydro furo[2,3-g] 1,4-oxazino[5,6-c] 4-propyl 5H-benzopyranne

0,81g du composé précédent sont traités par l'aluminohydrure de lithium comme décrit à l'exemple 1 stade F pour donner, après flash-chromatographie, 0,21g du composé désiré sous forme de base libre.
RMN (CDCl₃):

6,7 ppm, s, 2H ; 4,7 à 4,4 ppm, m,4H 4,1 ppm, d,1H ;3,9 ppm,m,2H; 3,0-3,3 ppm m,3H ; 2,8 à 2,5 ppm, m,4H ; 1,55 ppm m,2H ; 0,95ppm t,3H.

Ce produit est repris dans 10 ml d'éther, on y ajoute 0,4 ml d'éther chlorhydrique 2,3N (1,1 éq.), puis on filtre sur fritté le solide formé, le rince à l'éther et le sèche sous vide pour obtenir 0,22g du composé désiré sous forme de chlorhydrate.
P.F. : 122-125°C (MK) Rendement : 30%
RMN (DMSO-d6 + NaOD) :

6,65 ppm s, 1H ; 6,55 ppm s,1H; 4,5 à 4,3 ppm m,3H ; 4,3 ppm dd,1H; 4,0 ppm dd,1H; 3,65 ppm m,2H ; 3,1 ppm t,2H ; 2,85 ppm m,1H ; 2,7 à 2,4 ppm m,4H ; 1,45ppm m, 2H; 0,85 ppm t,3H.

### Exemple 5 :

### trans 3,4,4a,8,9,11b-hexahydro furo[2,3-g]1,4-oxazino[5,6-c] 4-propyl 5H-benzopyrane

### Stade A : 2,3,7,8-tétrahydro 3-propionylamino 4-oxo furo[2,3-g] benzopyrane

7,25g du produit obtenu à la préparation 3 sont traités comme décrit à l'exemple 1 stade D (en utilisant le chlorure de propionyle à la place du chlorure de 2-chloroacétyle) pour donner 7,3g du composé désiré.
P.F. : 184°C(K) Rendement : 94%

### Stade B : trans 2,3,7,8-tétrahydro 3-propionylamino 4-hydroxy furo[2,3-g] benzopyrane

6,5g du produit précédent sont traités par le borohydrure de sodium comme décrit à l'exemple 1 stade B pour donner 5,5g du composé désiré.
Rendement : 84%
RMN 400 MHz (DMSO-d6) :

7,7 ppm d,1H ; 6,7 ppm s, 1H ; 6,65 ppm s,1H ; 5,55 ppm d,1H ; 4,45 ppm t,2H ; 4,35 ppm m,1H ; 4,1 ppm m,1H ; 3,9 ppm m,2H ; 3,1 ppm m,2H ; 2,15 ppm m,2H ; 1,0 ppm t,3H.

Mise en évidence de l'isomérie trans par l'intermédiaire d'un effet Overhauser nucléaire entre les protons à 4,35 et 4,1 p.p.m.

### Stade C : trans 2,3,7,8-tétrahydro 3-propylamino 4-hydroxy furo[2,3-g] benzopyrane

5,4g du composé précédent sont traités par l'aluminohydrure de lithium comme décrit à l'exemple 1 stade C pour donner , après double recristallisation dans l'acétate d'éthyle, 2,8g du composé désiré.
P.F. : 136-138°C (K) Rendement : 55%

### Stade D : trans 2,3,7,8-tétrahydro 3-(N-propyl 2-chloroacétamido) 4-hydroxy furo[2,3-g] benzopyrane

2,7g du composé précédent sont traités par le chlorure de 2-chloroacétyle comme décrit à l'exemple 1 stade D pour donner 3,5g du composé désiré sous forme d'une meringue.
Rendement : 100%

### Stade E : trans 4a,8,9,11b-tétrahydro furo[2',3'-g] 1,4-oxazino[5,6-c] 3-oxo 4-propyl 2H,5H-benzopyrane

3,4g du composé précédent sont traités par l'hydrure de sodium comme décrit à l'exemple 1 stade E pour donner, après flash-chromatographie, 2,35g du composé désiré.
P.F. : 185°C (K) Rendement : 78%

### Stade F : trans 3,4,4a,8,9,11b-hexahydro furo[2,3-g] 1,4-oxazino[5,6-c] 4-propyl 5H-benzopyrane

2,25g du composé précédent sont traités par l'aluminohydrure de lithium comme décrit à l'exemple 1 stade F pour donner , après flash-chromatographie, puis recristallisation dans l'acétate d'éthyle, 0,16g du composé désiré sous forme de base libre.
P.F. : 117-119°C (K) Rendement : 7%
RMN (CDCl₃):

6,8 ppm s,1H ; 6,6 ppm s,1H ; 4,5 ppm m,3H ; 4,4 ppm d,1H (J≃ 9,2 Hz); 4,05 ppm dd,1H; 3,9 ppm dd,1H ; 3,85 ppm m, 1H ; 3,15 ppm t,2H; 2,9 ppm dd,1H; 2,7 ppm m,1H ; 2,4-2,6 ppm m,2H ; 2,25 ppm m, 1H ; 1,7 à 1,4 ppm m,2H ; 0,95 ppm t,3H.

### Exemple 6

### Trans 3,4,4a,11b-tétrahydro furo[2,3-g]1,4-oxazino[5,6-c]4-propyl 5H-benzopyrane

0,72 g (2,6 mmole) du composé titre de l'exemple 5 est dissous dans 100 ml d'acide acétique. On ajoute en une seule fois 20 ml d'eau puis par portions 2,8 g (7,8 mmole) de 2,3-dichloro 5,6-dicyano 1,4-benzoquinone. On porte 12 heures à reflux puis on ajoute 0,9 g (3,9 mmole) de 2,3-dichloro 5,6-dicyano 1,4-benzoquinone par fraction et porte de nouveau 10 heures à reflux. On laisse refroidir, évapore à sec et purifie par flash chromatographie sur silice (éluant : CH₂Cl₂/CH₃COOEt : 90/10). On obtient alors 0,17 g de produit attendu, dont le chlorhydrate fond à 200-205 °C.

### Exemple 7

### Trans 4-aza 1,2,3,4,4a,5,6,11b-octahydro 4-propyl furo[2,3-b]phénanthrène.

### Stade A : 9-méthoxy 1,4,5,6-tétrahydro benzo[f] quinolin-3-(2H)-one

A une solution de 25 g de 7-méthoxy-2-tétralone dans 285 ml de benzène et d'une quantité catalytique d'acide paratoluène sulfonique portée à reflux sont ajoutés goutte à goutte 15,6 g de pyrrolidine dans 145 ml de benzène. Après avoir obtenu la quantité d'eau théorique, le benzène est évaporé sous pression réduite et 62,4 g d'acrylamide sont ajoutés. Le mélange réactionnel est chauffé 90 minutes à 80 °C puis 30 minutes à 140 °C. Après refroidissement, le milieu est repris par du chlorure de méthylène, puis lavé à l'eau, séché sur sulfate de magnésium puis évaporé à siccité. Le résidu est recristallisé de l'acide acétique pour donner 10,3 g de produit attendu.
PF : 232-234 °C (K). Rendement : 32 %

### Stade B : trans 9-méthoxy 1,4,4a,5,6,10b-hexahydro benzo[f]quinolin-3-(2H)-one

On additionne 10,4 g de triéthylsilane à une solution de 7 g du composé obtenu au stade précédent dans 80 ml de dichlorométhane. Le mélange est agité 10 minutes à température ambiante. On ajoute ensuite 39 ml d'acide trifluoroacétique en refroidissant le mélange avec un bain de glace. Le mélange est agité pendant 18 heures à température ambiante. Après évaporation du solvant à l'évaporateur rotatif, on obtient une huile jaune qui est dissoute dans du dichlorométhane. La phase organique est lavée avec une solution d'hydrogénocarbonate de sodium jusqu'à un pH basique, séchée sur sulfate de magnésium et le solvant est évaporé à l'évaporateur rotatif. On reprend le résidu à l'hexane, décante et concréte dans l'acétonitrile. On obtient 6,3 g de l'isomère attendu.
PF : 222-224 °C (K). Rendement : 74 %.
RMN 400 MHz : (CDCl₃)
spectre ¹H :

7,1ppm (d, 1H); 6,85 ppm (dd, 1H); 6,75 ppm (dd, 1H); 3,8 ppm (s, 3H); 3,4 ppm (m,1H, J=11Hz) ; 2,9 ppm (m, 2H); 2,7 ppm (m, 1H, J=11Hz); 2,65 ppm (m, 3H); 2,1/1,9 ppm (m, 2H); 1,75 ppm (m, 1H); 7,7 ppm (s, 1H),J=11Hz : isomérie de jonction de cycle trans.

### Stade C : trans 9-méthoxy 1,2,3,4,4a,5,6,10b-octahydro benzo[f]quinoline

Une solution de 8,3 g du composé obtenu au stade B dans 83 ml de tétrahydrofurane est ajoutée goutte à goutte à une suspension de 6,2 g d'aluminohydrure de lithium. Après 18 heures de reflux, le mélange est hydrolysé par successivement 4,1 ml d'eau, 3,3 ml de soude à 20 %, 15,4 ml d'eau. Après filtration des sels minéraux, puis concentration du filtrat à l'évaporateur rotatif, on obtient 4,5 g du produit attendu.
PF : 68-70 °C (K). Rendement : 60 %.

### Stade D: trans 9-méthoxy 4-propionyl 1,2,3,4,4a,5,6,10b-octahydro benzo[f]quinoline.

A une solution de 4,5 g du composé obtenu au stade précédent dans 45 ml de dichlorométhane, on ajoute 3,7 ml de triéthylamine suivi de 1,8 ml de chlorure de propionyle à température ambiante. Le mélange est agité pendant 18 heures à température ambiante. Après évaporation du solvant à l'évaporateur rotatif, on reprend le résidu par de l'acétate d'éthyle, lave à l'eau, sèche sur sulfate de magnésium et concentre le solvant à siccité. Par purification par flash-chromatographie, on obtient 5 g du produit attendu.
Rendement : 86 %

### Stade E : trans 9-hydroxy 4-propionyl 1,2,3,4,4a,5,6,10b-octahydro benzo[f]quinoline.

On refroidit à 0 °C une solution de 6,8 g du composé obtenu au stade D dans 72 ml de chloroforme puis coule goutte à goutte 5,3 ml de tribromure de bore. On agite pendant 18 heures à température ambiante avant d'ajouter 20 ml d'éthanol. On filtre et lave à l'eau le précipité obtenu, 5,1 g du composé attendu sont ainsi isolés.
Rendement : 79 %.

### Stade F : trans 8-[(2-chloro-1-oxo)éthyl] 9-hydroxy 4-propionyl 1,2,3,4,4a,5,6,10b-octahydro benzo[f]quinoline.

47 ml d'une solution 1M de trichlorure de bore dans le dichlorométhane sont refroidis à 0 °C. On ajoute par portion, 5,1 g de composé décrit ci-dessus puis 3 ml de chloracétonitrile suivi de 2,6 g de chlorure d'aluminium anhydre. On laisse 4 heures sous agitation à 0 °C puis 18 heures à température ambiante avant d'hydrolyser successivement par 12,2 ml d'eau, 22 ml d'acide chlorhydrique à 10 % en refroidissant. On amène jusqu'à un pH basique avec de l'ammoniaque concentrée. On décante, extrait la phase aqueuse au dichlorométhane, lave à l'eau la phase organique et la séche sur sulfate de magnésium anhydre. Après évaporation du solvant sous vide, 5,9 g de produit attendu sont obtenus.
PF : 177-179 °C (K). Rendement : 89 %.

### Stade G : trans 4-aza-1,2,3,4,4a,5,6,11b-octahydro (9H) 4-propionyl furo[2,3-b]phénanthrèn-8-one.

On maintient sous agitation pendant 2 heures 30 à reflux 8,3 g du composé obtenu au stade précédent dans 142 ml de dichlorométhane et de 17,8 ml de triéthylamine. Après évaporation du solvant sous vide, le résidu est traité à l'eau et extrait à l'acétate d'éthyle. De la phase organique décantée, séchée sur sulfate de magnésium anhydre, 5 g du produit attendu sont obtenus.
PF : 172-176 °C (K). Rendement:95%.

### Stade H : trans 1-(8-hydroxy 1,2,3,4,4a,5,6,8,9,11b-décahydro -décahydro 4-aza furo[2,3-b]phénanthrèn-4-yl) propan-1-one.

A une solution de 4,9 g du composé obtenu précédemment dans 41 ml de méthanol, on ajoute 20 ml d'une solution d'hydrogénocarbonate de sodium à 10 % et 1,23 g de borohydrure de sodium, par portion, à température ambiante. Après évaporation du solvant à l'évaporateur rotatif, on reprend le résidu par de l'acétate d'éthyle, lave à l'eau, sèche sur sulfate de magnésium et concentre le solvant sous vide. 3 g du produit attendu sont obtenus.
Rendement : 61 %

### Stade I: trans 1-(1,2,3,4,4a,5,6,11b-octahydro -octahydro 4-aza furo[2,3-b]phénanthrén-4-yl)-propan-1-one.

On maintient sous agitation magnétique pendant 4 heures 3 g du composé obtenu précédemment dans 18,7 ml d'acide chlorhydrique à 10 %. On extrait au dichlorométhane, décante, lave à la soude N et sèche sur sulfate de magnésium anhydre. Après évaporation sous vide de la phase organique 2,3 g du produit attendu sont isolés.
Rendement : 81,5 %.

### Stade J : produit titre (sous forme de chlorhydrate)

On ajoute 1,66 ml (5,82 mmoles) de Red-Al® 3,5M dans du toluène sur 0,55 g (1,94 mmoles) du composé obtenu au stade I en solution dans 13,7 ml de toluène. On maintient l'agitation à température ambiante pendant 3 heures puis hydrolyse en ajoutant 0,75 ml d'éthanol puis 1 ml d'H₂O. On filtre les sels qui précipitent, extrait la phase toluénique par HCl N, basifie la phase aqueuse avec NaOH N puis l'extrait avec CH₂Cl₂. On sèche sur MgSO₄ et concentre.

L'huile obtenue est solubilisée dans 15 ml d'acétonitrile auquel on additionne 0,8 ml d'éther chlorhydrique 2,5 N, pour obtenir le chlorhydrate. On filtre le précipité forme. On obtient 0,35 g de chlorhydrate du produit titre.
PF : 235-238 °C (MK) Rendement : 59 %.
RMN (DMSO-d₆)
spectre ¹H :

11ppm, massif échangeable D₂O ; 7,95 ppm, d, 1H ; 7,5 ppm, s,1 H ; 7,4 ppm, s, 1H ; 6,85 ppm, d, 1H ; 3,5 ppm, d, 1H ; 3,4 à 2,9 ppm, m, 7H ; 2,6 ppm, m, 1H ; 2,4 ppm, m, 1H ; 2,2 à 1,9 ppm, m, 3H ; 1,7 ppm, m, 2H ; 1,5 ppm, m, 1H ; 1 ppm, t, 3H.

### Exemple 8

### cis 4-aza 1,2,3,4,4a,5,6,11b-octahydro 4-propyl furo[2,3-b] phénanthrène.

Le composé titre est obtenu de la même façon que le composé de l'exemple 7 mais en utilisant au stade C le cis 9-méthoxy-1,4,4a,5,6,10b-hexahydro benzo[f]quinolin-3-(*2H*)-one, obtenu de la manière suivante :

0,5 g du composé obtenu au stade A de l'exemple 7 est mis sous agitation dans 18 ml d'acide acétique avec 0,2 g de palladium sur charbon à 5 % à température ambiante sous 200 g de pression d'hydrogène pendant 18 heures. Après filtration du catalyseur et évaporation du solvant 0,5 g de résidu est chromatographié sur colonne de silice (éluant : CH₂Cl₂/CH₃COOEt ; 85/15). On obtient alors 0,32 g de dérivé cis.
Rendement : 62 %.
RMN 400 MHz : (CDCl₃)
spectre ¹H :

7,0 ppm (d,1H) ; 6,75 ppm (m,2H) ; 6,0 ppm (s,1H) ; 3,85 ppm (m,1H,J=5,5 Hz) ; 3,8 ppm (s,3H) ; 3,2/2,8 ppm (m, 6H) ; 3,15 ppm (m,1H,J=5,5 Hz) ; 2,1 ppm (m,2H).J=5,5 Hz : isomérie de jonction de cycle cis.

### Préparation des matières premières nouvelles

### Préparation 1 : 2,3,7,8-tétrahydro 4-oxo furo [2,3-g] benzopyrane

### Stade A : 3-(2,3-dihydro benzofuran-5-yloxy)propionitrile

A température ambiante, on mélange 40,8g de 2,3-dihydro 5-hydroxy benzofuranne (dont la synthèse est décrite dans Synthesis 1988, 950-952), 3 ml d'une solution de Triton B à 40% dans le méthanol et 200 ml d'acrylonitrile fraichement distillé. On porte à reflux pendant 46h, puis évapore au maximum l'acrylonitrile. Le résidu est repris à l'acétate d'éthyle, lavé à la soude 2N, à l'acide chlorhydrique N et à l'eau. La phase organique, séchée sur sulfate de magnésium, est concentrée sous vide, puis le résidu est recristallisé dans 300 ml d'isopropanol pour donner 38g du composé désiré.
P.F. <50°C (K) Rendement : 67%

### Stade B : acide 3-(2,3-dihydro benzofuran-5-yloxy) propionique

On porte à reflux, pendant 5h, 10,6g du composé précédent et 21 ml d'acide chlorhydrique concentré. Après avoir refroidi, on extrait au dichlorométhane. Les phases organiques jointes sont lavées à l'eau, puis extraites par une solution aqueuse saturée d'hydrogénocarbonate de sodium. Les phases aqueuses basiques sont acidifiées à froid par de l'acide chlorhydrique concentré, on filtre le solide formé, le rince à l'eau et le sèche sous vide pour obtenir 9,65g du composé désiré.
P.F. : 131-132°C (K) Rendement : 82%

### Stade C : 2,3,7,8-tétrahydro 4-oxo furo[2,3-g] benzopyranne

A 46,5g d'anhydride phosphorique dans 465 ml d'acide méthanesulfonique à 60°C, on ajoute 34,1g du composé précédent et agite pendant 10 mn à 60°C. On verse alors dans 2 l d'un mélange eau/glace, et extrait à l'éther. Les phases organiques jointes sont lavées à la soude N et à l'eau, puis séchées sur sulfate de magnésium et concentrées. Le résidu est recristallisé dans 80 ml d'éthanol pour donner 20,9g du composé désiré.
P.F. : 101°C(K) Rendement : 67%

### Préparation 2 :2,3,7,8-tétrahydro 3-azido 4-oxo furo [2,3-g] benzopyranne

### Stade A : 2,3,7,8-tétrahydro 3-bromo 4-oxo furo[2,3-g] benzopyranne

A 27g du composé titre de la préparation 1 dans 1400 ml de dichlorométhane et 560 ml de méthanol à température ambiante, on ajoute par fractions 69,3g de tribromure de tétra-n-butyl ammonium. Après 5h sous agitation à température ambiante, on évapore les solvants, reprend au dichlorométhane et lave à l'acide chlorhydrique 2N et à l'eau. Après séchage sur sulfate de magnésium et évaporation du solvant, on obtient 38g du composé désiré, qui est utilisé tel quel.
Rendement : 100%

### Stade B : 2,3,7,8-tétrahydro 3-azido 4-oxo furo[2,3-g] benzopyranne

Au produit précédent dans 185 ml de DMF à température ambiante, on ajoute par fractions 12g d'azoture de sodium. Après 3 h d'agitation à température ambiante, on verse dans 2 l d'eau et extrait au dichlorométhane. Les phases organiques jointes sont lavées à l'eau, séchées sur sulfate de magnésium, puis concentrées pour donner 31g du composé désiré, qui est utilisé sans purification du fait de son instabilité.

### Préparation 3 : 2,3,7,8-tétrahydro 3-amino 4-oxo furo [2,3-g] benzopyranne

### Stade A : 7,8-dihydro 4-hydroxyimino furo[2,3-g] benzopyranne

27,4g de 2,3,7,8-tétrahydro 4-oxo furo[2,3-g] benzopyranne (voir préparation 1), 42g de chlorhydrate d'hydroxylamine et 41,9g d'acétate de sodium dans 288ml d'éthanol sont portés à reflux pendant 1 h. On évapore le solvant, reprend au dichlorométhane et lave à l'eau. Après séchage sur sulfate de magnésium, évaporation, puis recristallisation dans l'éthanol, on obtient 21,85g du composé désiré.
P.F. : 160°C(K) Rendement :74%

### Stade B : 2,3,7,8-tétrahydro 4-p-toluènesulfonyloxyimino furo[2,3-g] benzopyranne

A 21,8g du produit précédent dans 106 ml de pyridine à 0°C, on ajoute par fractions 24,3g de chlorure de tosyle. On agite ensuite 2 h à 0°C, puis 24 h à température ambiante. On verse alors dans 1 l d'eau et extrait à l'éther. Les phases éthérées jointes sont lavées à l'eau, à l'acide sulfurique 0,5N et à l'eau, puis séchées sur sulfate de magnésium et concentrées sous vide pour donner 36g du composé désiré.
P.F. : 118°C(K) Rendement : 94%

### Stade C : 2,3,7,8-tétrahydro 3-amino 4-oxo furo[2,3-g] benzopyranne

A de l'éthylate de sodium dans l'éthanol (préparé à partir de 2,64g de sodium et 100 ml d'éthanol anhydre) à 0°C, on ajoute 35,9g du produit précédent dans 130 ml de benzène. Après 6 h sous agitation à température ambiante, puis repos la nuit au réfrigérateur, on filtre le solide formé et le rince au benzène. Les filtrats sont versés sur 250 ml d'acide chlorhydrique 4N sous agitation énergique. Le solide formé est filtré et séché sous vide pour donner 18,7g du composé désiré sous forme de chlorhydrate.
P.F. > 260°C(K) Rendement : 78%

### Exemple 9: Etude pharmacologique.

La sélectivité pour les récepteurs D₃ vis à vis des récepteurs D₂ a été démontrée :
in vitro : par la technique du binding sur récepteurs D₂ et D₃ humains et de rats (exprimés indépendamment et de façon stable dans les cellules CHO) ;
in vivo : par la capacité des molécules à moduler l'hypothermie induite chez le rat par l'agoniste dopaminergique D₃ , 7-OH-DPAT, le contrôle de la température corporelle étant sous la dépendance du récepteur D₃ post-synaptique (M.J. Millan, op. cit.).

Les propriétés thérapeutiques et notamment celles anti-dépressives ont été démontrées en utilisant le test de Porsolt (test de la nage forcée).

### A - Selectivite D₃ vs D₂

1. Matériel et Méthode.
1.1 In vitro- Binding.
   L'affinité des composés vis à vis des récepteurs D₃ et D₂ humains ou de rats (exprimés indépendamment et de façon stable dans des cellules CHO) a été déterminée sur des préparations membranaires en utilisant le [¹²⁵I]-iodosulpiride comme radioligand (Sokoloff et al, o.p. cited), le raclopride (10 µM) déterminant la liaison non spécifique. Les résultats sont exprimés en IC₅₀. La sélectivité est exprimée par le rapport IC₅₀ D₂ / IC₅₀ D₃.
1.2. In vivo - Hypothermie chez le rat.
   Les tests ont été réalisés sur des rats Wistar, mâles, de 200-250 g, placés en cage individuelle avec libre accès à la nourriture et à l'eau. Les produits ont été solubilisés dans de l'eau distillée, à laquelle plusieurs gouttes d'acide lactique sont ajoutées. Les injections ont été effectuées dans un volume de 1.0 ml / kg par voie sous-cutanée. Les doses sont exprimées en terme de base. La température rectale des rats a été enregistrée en utilisant un thermistoprobe digital (Millan et al., J.P.E.T., 1993, 264, p 1364-1376). Dans un premier temps, les rats ont été injectés avec le composé ou le véhicule, puis replacés dans leurs cages pendant 30 minutes. Puis, les rats ont subi une injection de 7-OH-DPAT (0.16 mg / kg) et ont été replacés dans leurs cages. Trente minutes plus tard, la température rectale a été mesurée et la différence a été déterminée par rapport aux valeurs basales (Δ T°C). La Dose Inhibitrice (95% Limites de Confiance) pour réduire à 50% l'effet du 7-OH-DPAT a été calculée selon la méthode de Finney (Statistical Method in Biological Assays, 2^{nd} ed, Hafner Publishing, New-York, 1964).

2. Résultats
2.1. Binding
Les affinités (IC₅₀) des produits de l'invention pour les récepteurs D₃ sont comprises entre 10⁻⁹ M et 10⁻⁷ M, tandis que celles pour les récepteurs D₂ sont comprises entre 10⁻⁷ M et 10⁻⁵ M.
A titre d'exemple, la selectivité pour les récepteurs D₃ vis à vis des récepteurs D₂ exprimée par le rapport IC₅₀ D₂ / IC₅₀ D₃ est de 104 pour les récepteurs clonés de rat et de 45 pour les récepteurs clonés humains dans le cas du produit de l'exemple 1. Ces valeurs se comparent favorablement à celles obtenues avec l'agoniste spécifique 7-OH-DPAT pour lequel la selectivité D₃ vs D₂ est de 53 pour les récepteurs clonés de rat et surtout avec celles obtenues pour les antagonistes AJ 76 et (+)UH 232 dont la selectivité n'est respectivement que de 2,2 et de 4,8 pour les récepteurs clonés humains.
2.2. Hypothermie chez le rat.
L'effet des produits de l'invention au niveau des récepteurs D₃ in vivo est illustré par le comportement du composé de l'exemple 1 dans le modèle de l'hypothermie. Les valeurs obtenues au cours de ce test sont rapportées dans le tableau 1.

**Tableau 1**

| **INJECTION 1** | **INJECTION 2** | **ΔT°C** ^{**a)**} |
|---|---|---|
| Véhicule - | - | + 0,6 ± 0,1 |
| Véhicule - | 7-OH-DPAT (0,16 mg / kg) | -1,4 ± 0,1 |
| Ex 1 (0,16 mg / kg) | 7-OH-DPAT (0,16mg / kg) | -1,8 ± 0,2 |
| Ex 1 (0,63 mg / kg) | 7-OH-DPAT (0,16 mg / kg) | -1,3 ± 0,2 |
| Ex 1 (2,5 mg / kg) | 7-OH-DPAT (0,16 mg / kg) | +0,2 ± 0,2* |
| Ex 1 (10,0 mg / kg) | 7-OH-DPAT (0,16mg / kg) | -0,1 ± 0,2* |

| | | |
|---|---|---|
| a) les valeurs sont les moyennes ± S.E.M. N≥5 - 9 par valeur. | | |
| * p< 0,05 versus véhicule / 7-OH-DPAT selon le test de Dunnett. | | |

La dose inhibitrice 50 (ID₅₀) (95% L.C. = 95% de Limites de Confiance ) est de 1,6 (0,7 - 3,7) mg / kg s.c. Ceci met clairement en évidence que les produits de l'invention, non seulement reconnaissent in vitro les récepteurs D₃ mais agissent, in vivo, par l'intermédiaire de ces mêmes récepteurs D₃.

### B- Modèle thérapeutique

1- Materiel et méthode
Test de la nage forcée.
La procédure a été décrite en détail par Porsolt et al. (1978).
L'expérience a été effectuée en deux jours, le test ayant lieu le second jour. Le premier jour, l'animal a été placé pendant 15 minutes dans un cylindre de verre (30 cm x Ø 20 cm) rempli d'eau dont la température est maintenue à 25°C. Le second jour, jour du test, le produit ou le solvant a été administré à l'animal, par voie sous-cutanée, 30 minutes avant le début du test. A T₀, l'animal a été placé dans le cylindre rempli d'eau, pendant les 5 minutes que dure le test. Puis le temps total (exprimé en secondes) d'immobilité de l'animal a été mesuré.
La dose inhibitrice (95% Limites de Confiance) pour réduire de 50% le temps d'immobilité a été calculée selon la méthode de Finney (Statistical Method in Biological Assays, 2^{nd} ed., Hafner Publishing, New York, 1964).
2- Résultats
Pour illustrer l'effet anti-dépresseur revendiqué pour les produits de l'invention, sont rapportés ci-après les résultats obtenus avec l'un des composés représentatifs de l'invention.(cf Tableau 2). Le composé de l'exemple 1 montre dans ce test une dose inhibitrice 50 (ID₅₀) (95% L.C. = 95% de Limites de Confiance) de 1,8 (1,2 - 2,7) mg / kg s.c., révélant ainsi un puissant effet anti-dépresseur.

**Tableau 2**

| **Produit** | **Dose (mg / kg)** | **Temps d'immobilité (sec)** |
|---|---|---|
| Véhicule | | 188,0 ± 15,6 |
| Ex 1 | 0,63 | 200,8 ± 17,4 |
| Ex 1 | 1,25 | 107,1 ± 39,7 |
| Ex 1 | 2,5 | 63,1 ± 12,4* |
| Ex 1 | 10,0 | 28,2 ± 18,1* |

| | | |
|---|---|---|
| * p < 0.05 versus véhicule / 7-OH-DPAT selon le test de Dunnett. Les valeurs sont les moyennes ± S.E.M. N ≥ 4-7 par valeur. | | |

## Revendications

1. Les composés de formule **I**. 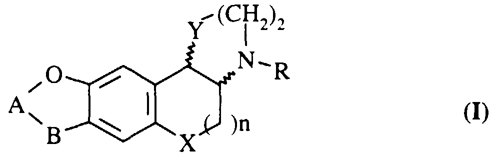 dans laquelle
- X et Y, identiques ou différents, représentent chacun un atome d'oxygène ou CH₂ ;
- A-B représente -(CH₂)₂- ou -HC=CH-, et de plus :
. lorsque Y représente un atome d'oxygène, A-B peut aussi représenter -(CH₂)₃- et
. lorsque Y représente CH₂, A-B peut aussi représenter 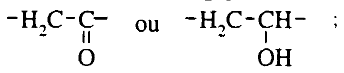
- R représente un atome d'hydrogène ou un radical (C₁-C₁₀) alkyle, (C₃-C₁₀) alkényle ou (C₃-C₁₀) alkinyle, chacun pouvant être en chaîne droite ou ramifiée et chacun pouvant être substitué par un radical cycloalkyle ayant de 3 à 8 atomes de carbone, ou par un radical aryle choisi parmi les radicaux phényle, thiényle et pyridyle, chacun d'eux éventuellement substitué par un ou plusieurs substituants choisis parmi les atomes d'halogène le radical hydroxy et les radicaux alkyle et alkoxy ayant chacun de 1 à 6 atomes de carbone en chaîne droite ou ramifiée ; et
- n représente :
. zéro ou 1 lorsque X représente -CH₂ et
. uniquement 1 lorsque X représente un atome d'oxygène.
- leurs formes isomères géométriques cis et trans,
- leurs formes de mélange racémique ou racémate et d'isomères optiques ou énantiomères,
ainsi que leurs sels avec des acides pharmaceutiquement acceptables.

2. Un composé selon la revendication 1 qui est :
le trans 3,4,4a,5,6,8,9,11b-octahydrofuro[2,3-b]1,4-oxazino[3,2h] 4-propyl-2H-naphtalène.

3. Un composé de la revendication 1 qui est :
le trans 4-aza 1,2,3,4,4a,5,6,11b-octahydro 4-propyl furo[2,3-b]phénanthrène.

4. Les compositions pharmaceutiques utilisables dans le traitement des états psychotiques, de la dépression, de la maladie de Parkinson, des troubles mnésiques et des troubles liés à l'abus de drogue, contenant comme principe actif un composé selon une des revendications 1 à 3, avec un ou plusieurs excipients pharmaceutiques appropriés.

## Patentansprüche

1. Verbindungen der Formel I: 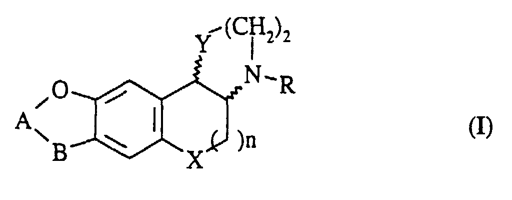 in der
- X und Y, die gleichartig oder verschieden sind, Jeweils ein Sauerstoffatom oder CH₂ bedeuten;
- A-B -(CH₂)₂- oder -HC=CH- bedeuten und darüber hinaus:
. wenn Y ein Sauerstoffatom darstellt, A-B auch -(CH₂)₃- bedeuten kann und
. wenn Y CH₂ darstellt, A-B auch 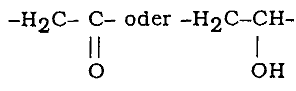 bedeuten kann;
- R ein Wasserstoffatom oder eine (C₁-C₁₀)-Alkylgruppe, (C₃-C₁₀)-Alkenylgruppe oder (C₃-C₁₀)-Alkinylgruppe bedeutet, wobei Jede dieser Gruppen eine gerade oder verzweigte Kette aufweist und jeweils substituiert sein kann durch eine Cycloalkylgruppe mit 3 bis 8 Kohlenstoffatomen oder eine Arylgruppe ausgewählt aus Phenyl-, Thienyl- und Pyridylresten, wobei Jeder dieser Reste gegebenenfalls durch einen oder mehrere Substituenten ausgewählt aus Halogen, Hydroxygruppen und Alkyl- und Alkoxygruppen mit 1 bis 6 Kohlenstoffatomen in gerader oder verzweigter Kette substituiert sein kann; und
- n:
. Null oder 1, wenn X -CH₂ darstellt und
. lediglich 1, wenn X ein Sauerstoffatom darstellt, bedeutet,
- deren geometrische cis- und trans-Isomere,
- deren racemische Mischung oder Racemate und optische Isomere oder Enantiomere
sowie deren Additionssalze mit pharmazeutisch annehmbaren Säuren.

2. Verbindung nach Anspruch 1, nämlich:
trans-3,4,4a,5,6,8,9,11b-Octahydrofuro[2,3-b]-1,4-oxazino[3,2-h]-4-propyl-2H-naphthalin.

3. Verbindung nach Anspruch 1, nämlich:
trans-4-Aza-1,2,3,4,4a,5,6,11b-octahydro-4-propyl-furo[2,3-b]phenanthren.

4. Pharmazeutische Zubereitungen zur Behandlung von psychotischen Zuständen, der Depression, der Parkinsonschen Krankheit, von Gedächtnisstörungen und Störungen, die mit dem Drogenmißbrauch verknüpft sind, enthaltend als Wirkstoff eine Verbindung nach einem der Ansprüche 1 bis 3 zusammen mit einem oder mehreren pharmazeutisch geeigneten Trägermaterialien.

## Claims

1. Compounds of formula **I** 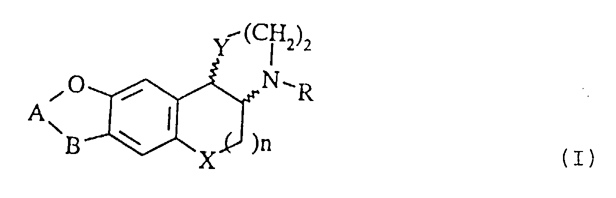 wherein :
- X and Y, which may be the same or different, each represents an oxygen atom or CH₂;
- A-B represents -(CH₂)₂- or -HC=CH- and, in addition :
. when Y represents an oxygen atom, A-B may also represent -(CH₂)₃- and
. when Y represents CH₂, A-B may also represent 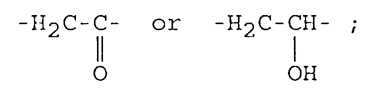
- R represents a hydrogen atom or a (C₁-C₁₀)alkyl, (C₃-C₁₀)alkenyl or (C₃-C₁₀)alkynyl radical, each of which may be in straight or branched chain and each of which may be substituted by a cycloalkyl radical having from 3 to 8 carbon atoms, or by an aryl radical selected from the radicals phenyl, thienyl and pyridyl, each of which may optionally be substituted by one or more substituents selected from halogen atoms, hydroxy radicals and alkyl and alkoxy radicals each having from 1 to 6 carbon atoms in straight or branched chain; and
- n represents :
. 0 or 1 when X represents CH₂ and
. 1 only, when X represents an oxygen atom.
- their geometric isomer forms, cis and trans,
- their racemic mixture or racemate and optical isomer or enantiomer forms,
and also salts thereof with pharmaceutically acceptable acids.

2. A compound according to claim 1 which is :
trans-3,4,4a,5,6,8,9,11b-octahydrofuro[2,3-b]1,4-oxazino-[3,2-h]4-propyl-2H-naphthalene.

3. A compound according to claim 1 which is :
trans-4-aza-1,2,3,4,4a,5,6,11b-octahydro-4-propyl-furo[2,3-b]phenanthrene.

4. Pharmaceutical compositions that can be used in the treatment of psychotic conditions, depression, Parkinsons disease, memory disorders and disorders associated with drug abuse, comprising as active ingredient a compound according to any one of claims 1 to 3 with one or more appropriate pharmaceutical excipients.
